# EUROPEAN PATENT APPLICATION

(11) **EP 0 627 220 A1**
(43) Date of publication of application: **07.12.1994**
(21) Application number: 94108474.1
(22) Date of filing: 01.06.1994
(51) Int. Cl.: A61K 31/16, A61K 9/02, A61K 9/48

(54) **Pharmaceutical compositions for the treatment of thalassemia**

(30) Priority: 04.06.1993 IT MI931174
(71) Applicant: EMERGING PHARMACEUTICAL TECHNOLOGIES INC., Sugar Land, Texas 77478 (US)
(72) Inventor: Hayhurst, Walter, Chicago, IL 60604 (US); Allen, Loyd, Chicago, IL 60604 (US); Girondi, Pat, Chicago, IL 60604 (US)
(74) Representative: Bianchetti, Giuseppe

(57) **Abstract**

Pharmaceutical compositions containing butyric acid derivatives, particularly isobutyramide, in the form of capsules and suppositories, are advantageously indicated for the therapy of thalassemia when compared with known formulations.

## Description

The object of the present invention consists of pharmaceutical compositions for the treatment of thalassemia, containing butyric acid derivatives as active ingredient, particularly isobutyramide, said compositions being in the form of capsules and suppositories.

It is known from EP 0 371 789 and from US 4,822,821 that butyric or isobutyric acid and their derivatives can be used for the therapy of the clinical effects of β-globin disorders and in particular of thalassemiae. Under this name are grouped chronic, family-, haemolytic anaemias, occurring in certain geographical areas, particularly in the coastal Mediterranean countries, it is also referred as "Mediterranean anaemia", which is characterized by the production of abnormally thin erythrocytes, which show an accentuated mechanical fragility. Etiology of this disorder involves a reduced synthesis of the hemoglobin β- or α- polypeptide chain, and an insufficient erythropoiesis. The symptoms are those of a severe anaemia: jaundice, leg ulcers, cholelithiasis. Splenomegaly is frequent, so that spleen can become enormous. A collapse of the erythrocyte number occurs, and also the mean corpuscular volume is abnormously low. Few patients survive beyond puberty.

According to the above mentioned European and United States patents, moderate therapeutical results can be obtained by administering butyric acid and its salts, α-aminobutyric acid, B-chloro-D-alanine, isobutyric acid, isobutyramide and others. Although said references mention the possibility to administer the active ingredient in capsules (or tablets), and other oral forms, it appears that the treatments are practically carried out by introducing the medicament directly in the blood stream (see EP 0 371 789, pag. 3, line 20 and following).

It has now been found that therapeutically more encouraging results are obtained by administering the butyric acid derivatives, and particularly the isobutyramide, in the form of capsules or suppositories. The unitary doses of active ingredient (preferably isobutyramide) are comprised between 100 and 500 mg for capsules, and between 500 and 1,500 mg for suppository. In the case of capsules a controlled release form, wherein the active ingredient is admixed with a controlled release hydrophilic matrix, proved to be particularly effective.

The suppositories can contain a base consisting of common fatty acids or polyethylene glycol.

The daily dosage can be such as to provide about 1.2 g of active ingredient (preferably isobutyramide) within 24 hours, when the administration is performed orally; in the case of suppository use, the amount also can markedly exceed the above mentioned one, and reach, for example, about 1,800-2,000 mg/day.

It is also provided the administration every other day, suitably with twice the amount than the above stated one.

The following examples further illustrate the invention.

### EXAMPLE 1

Isobutyramide capsules.

Content for each capsule:

| | |
|---|---|
| Isobutyramide | 200 mg |
| Methylcellulose ("premium", controlled release excipient) | 200 mg |

Finely powdered isobutyramide crystals were admixed with excipient and introduced in capsules in the above mentioned amount. The dosage provides 2 capsules three times a day, daily, or 4 capsules three times a day, every other day.

Methylcellulose "premium" type is a cellulose ether prepared in a special way for pharmaceutical uses.

It is a controlled release hydrophilic matrix which gives rise to a dynamic system consisting of the wetting, hydration and dissolution of the polymer. In the hydration phase, isobutyramide slowly dissolves and migrates through the matrix made very viscous.

### EXAMPLE 2

Isobutyramide suppositories.

Each suppository consists of:

| | |
|---|---|
| Isobutyramide | 0.65 g |
| Fatty acids base mass for suppositories | 1.35 g |

The mass was melted in a water-bath and finely powdered isobutyramide was added under stirring. The mixture was slowly cooled until a viscous mass was obtained, such that however to allow the mass itself to be poured into the mould as to fill it uniformly. After refrigerating for about one hour, the suppositories were removed and packaged.

### EXAMPLE 3

Suppositories.

Each suppository consists of:

| | |
|---|---|
| Isobutyramide | 0.65 g |
| Polyethylene glycol (PEG) base mass for suppositories | 1.35 g |

The PEG mass was melted in a water-bath at about 55°C, and finely powdered isobutyramide was added under stirring. After thorough mixing, the mixture was cooled until a viscous mass was obtained, but sufficiently fluid to be poured into the mould as to fill it uniformly. After refrigerating, the suppositories were packaged.

The suppositories of the Examples 2 and 3 are physically stable also at room temperature.

Formulations similar to those described in the Examples 1-3 are obtained using arginine (iso)butyrate and sodium (iso)butyrate as active principles.

## Claims

1. Pharmaceutical compositions containing butyric and/or isobutyric acid and/or derivatives thereof, for the therapy of thalassemia, characterized in that said compositions are in the form of capsules or suppositories.

2. Pharmaceutical compositions according to claim 1, characterized in that they contain isobutyramide as active ingredient.

3. Capsules according to claims 1 and 2, containing controlled release methylcellulose "premium" type as excipient.

4. Suppositories according to claims 1-2, containing a fatty acids base mass for suppositories.

5. Suppositories according to claims 1-2, containing a polyethylene glycol base mass for suppositories.
